# EUROPEAN PATENT SPECIFICATION

(11) **EP 1 656 842 B1**
(45) Date of publication and mention of the grant of the patent: **23.01.2008**
(21) Application number: 04742070.8
(22) Date of filing: 21.07.2004
(51) Int. Cl.: A24F 47/00, A24D 1/00, A24D 3/00

(54) **SIMULATED CIGARETTE WHICH IS DESIGNED TO HELP QUELL TOBACCO ADDICTION**
ZUM EINDÄMMEN DER TABAKSUCHT ENTWICKELTER ZIGARETTENERSATZ
IMITATION DE CIGARETTE DESTINEE A FAVORISER L'ELIMINATION DE L'ADDICTION AU TABAC

(30) Priority: 24.07.2003 ES 200301783 U
(43) Date of publication of application: 17.05.2006
(73) Proprietor: Martinez Fernandez, Jose Antonio, 36579 Lamela-Silleda (ES)
(72) Inventor: Martinez Fernandez, Jose Antonio, 36579 Lamela-Silleda (ES)
(74) Representative: Munoz Garcia, Antonio
(86) International application number: PCT/ES2004/000342
(87) International publication number: WO 2005/009152

(56) References cited:
- US-A- 2 342 853
- US-A- 3 320 953
- US-A- 3 521 643
- US-A- 4 083 372
- US-A- 4 429 703
- US-A- 4 995 407
- US-A1- 2004 003 820

## Description

### OBJECT OF THE INVENTION

The present specification refers to a simulated cigarette which is designed to help quell tobacco addiction, the evident purpose of which is to eliminate, reduce or at least palliate addiction to tobacco, being made up of a body similar to a standard cigarette and which when inhaling through it has a small resistance to air, similar to the resistance created from the inhalation carried out with a standard cigarette and at the same time and in a parallel manner, it provides the consumer with a fresh and pleasant taste that decreases or reduces anxiety resulting from a lack of nicotine.

### FIELD OF THE INVENTION

This invention is applicable in the industry dedicated to manufacturing auxiliary elements to reduce drug addiction habits.

### BACKGROUND OF THE INVENTION

The applicant is aware of the great difficulty that any habitual tobacco consumer has when attempting to quit this habit which creates tobacco addiction.

In the attempt to aid tobacco consumers, there are currently different chemical products which, presented as patches or as pills, attempt to aid the consumer to lose the state of addiction which he or she is in.

However like others, the applicant has found that these chemical products only partially comply with or cover the task, since it has been shown that the overwhelming majority of smokers not only crave the direct effects of tobacco on their system due to the lack of consumption, but they greatly crave and miss the mechanics of the act of smoking.

In this sense, the existence of some documents describing cigarettes that are similar to the one herein proposed is known, including US patent document 4,995,407, US patent document 3, 521, 643, US patent document 3,320,953, US patent document 4,429,703 and US patent document 2,342,853, which are essentially different from the cigarette herein proposed despite having some equivalent features, such as the fact that they are formed by rigid tubular bodies and include natural essences, providing the user with a false cigarette to help quell his/her addiction.

### DESCRIPTION OF THE INVENTION

The simulated cigarette which is designed to help quell tobacco addiction proposed by the invention is configured as a highly important element or product as it combines therein the effects demanded from the simulated cigarette, since on one hand it allows maintaining said manual mechanics that is a habit for smokers, while on the other hand it replaces tobacco smoke due to the inhalation of the duly combined essences generated from the simulated cigarette, which eliminate or quell a smoker's craving.

More specifically, the simulated cigarette which is designed to help quell tobacco addiction object of the invention is made up of a tubular or cylindrical body made from a rigid material, presented in the form of a tube in which two areas with different diameters are arranged, the one with the larger diameter, and in turn longest in length, having the purpose of being used as an internal housing for the tobacco replacement products, whereas the area having a smaller diameter is intended for externally housing a tip made of a soft material which is both visually and mechanically equivalent to the tip of a conventional cigarette, this assembly configured by means of the body, tip and their outer features adopting the general shape of a conventional cigarette.

A filter is arranged in the area having the larger diameter of said body forming the simulated cigarette, after its inner end, with the function of achieving that the person who inhales obtains the essences and the flavor of the products existing inside the recipient where they are housed, thus preventing the product form reaching the consumer in solid form, effecting and assuring the fact of being presented as a product lasting between one and two weeks, and then the product is added, which is made up of a menthol part assuring freshness upon inhalation, valerian, which reduces anxiety created by the lack of nicotine, and then the remaining elements making up the cigarette can have different flavors, such as mint, strawberry, etc., so that the consumer will always have the flavor he or she most likes in the simulated cigarette, and therefore making it more comfortable to replace tobacco.

### DESCRIPTION OF THE DRAWINGS

To complement the description being made and for the purpose of aiding to better understand the features of the invention, a set of drawings is attached to the present specification as an integral part thereof, in which the following is shown with an illustrative and non-limiting character:
Figure 1 shows a perspective view of the object of the invention corresponding to a simulated cigarette to help quell tobacco addiction.
Figure 2 shows an exploded perspective view of the body of the cigarette, the tip and plug forming the object shown in Figure 1.

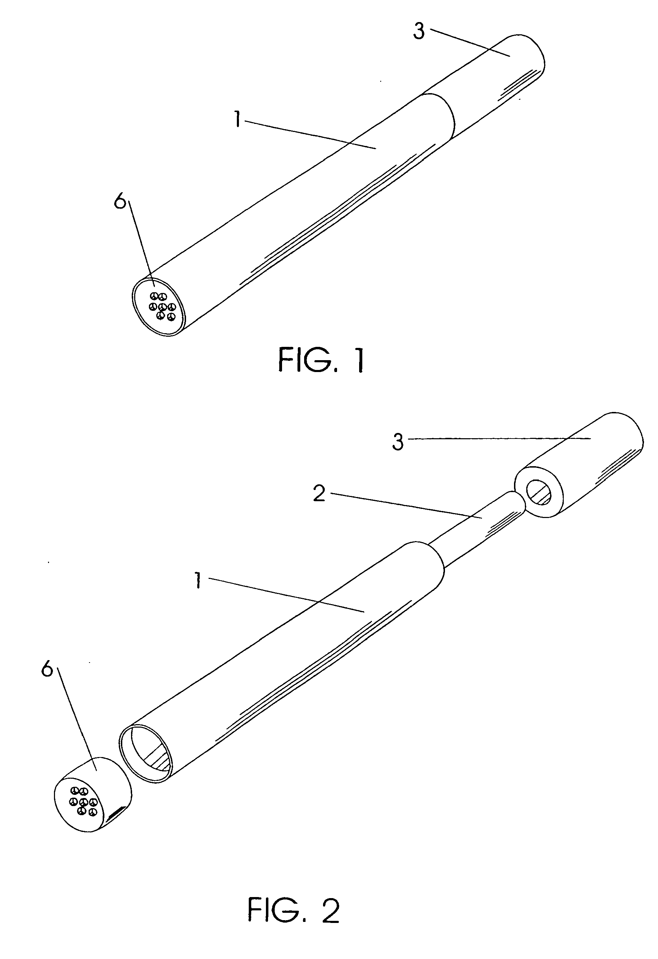

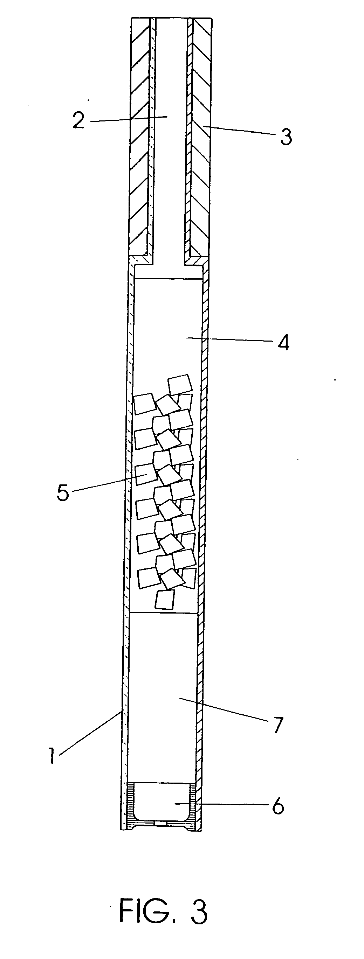

Finally Figure 3 shows a sectional view of the object shown in Figure 1, this graphic representation showing the arrangement of the different components forming it.

### PREFERRED EMBODIMENT OF THE INVENTION

In view of these figures it can be seen how the simulated cigarette which is designed to help quell tobacco addiction is made up of a tubular body (1) made from a rigid material such as a plastic material, in which two areas having different diameters are arranged, one having a smaller diameter and smaller axial height which is configured as an extension of the body (1) and is marked with reference number (2), forming a neck intended for externally receiving a tip (3) located on its outer end, made from a soft material, with an axial height coinciding with the axial height of the area (2) and an outer diameter in turn coinciding with the general body (1), such that after assembling the tip (3) on the general body, this assembly has a configuration and dimensions similar to those of a standard cigarette with a tip.

Arranged inside the body (1), specifically in the central area thereof, there is a filter (4) allowing the passage of air through it, retaining the remaining components of the simulated cigarette, specifically the product (5) made up of a mixture of menthol, valerian and a flavor chosen for this unit, and closing the assembly of the area (1) by means of a plug (6) made from a rigid material, provided with perforations allowing the passage of air through it.

The incorporation of another filter (7) has been provided in the area (1) close to the area provided with the plug (6) for the purpose of achieving that the mixture carried out with menthol, valerian and the flavoring agent forming the essences (5) may be open so that the components in question perform the appropriate modification of volumes so as to obtain a stronger or smoother flavor of the simulated cigarette.

## Claims

1. A simulated cigarette which is designed to help quell tobacco addiction, of the type formed by a tubular body (1) made from a rigid material, preferably plastic, internally incorporating a first filter (4) and natural essences (5), comprising a tip (3) and a plug (6), **characterized by** the fact that the mentioned tubular body (1) comprises two sections having different diameters, one with a smaller diameter and a smaller axial height, configured as an extension of the body (1) forming a neck intended to externally receive the tip (3) located in its outer area, made from a soft material, having an axial height matching the axial height of area (2) and having an outer diameter in turn matching the general body (1), such that after assembling the tip (3) on the general body, this assembly adopts a configuration and dimensions that are similar to those of a conventional cigarette with a tip; and by the fact that the incorporation of a second filter (7) has been provided in the area of the tubular body (1) close to the plug (6) .

## Patentansprüche

1. Simulierte Zigarette zur Begünstigung der Beseitigung der Tabaksucht, von der aus einem röhrenförmigen Körper (1) bestehenden Art, aus starrem Material hergestellt, vorzugsweise aus Kunststoff, in dessen Inneren er einen Filter (4) und Naturessenzen (5) eingliedert, wobei der ein Mundstück (3) und einen Verschluss (6) umfasst, **gekennzeichnet durch** die Tatsache, dass der erwähnte röhrenförmige Körper (1) zwei Bereiche von unterschiedlichem Durchmesser umfasst, einen von einem kleineren Durchmesser und geringerer Axialhöhe, der sich als eine Verlängerung des Körpers (1) gestaltet, wobei der einen um das in seiner Außenzone gelegenes Mundstück (3), aus weichem Material hergestellt, äußerlich aufzunehmen vorgesehenen Hals bildet, von mit der Axialhöhe der Zone (2) übereinstimmender Axialhöhe und von äußerem Durchmesser, seinerseits übereinstimmend mit dem Hauptkörper (1), so dass nach dem Einbau des Mundstücks (3) auf dem Hauptkörper dieser Satz eine einer konventionellen Zigarette mit Mundstück ähnliche Gestaltung und Größe annimmt; und aufgrund der Tatsache, dass man in der dem Verschluss (6) nächstgelegenen Zone des röhrenförmigen Körpers (1) die Eingliederung eines weiteren Filters (7) vorgesehen hat.

## Revendications

1. Fausse cigarette pour favoriser l'élimination de l'addiction au tabac, du type constitué à partir d'un corps tubulaire (1) fabriqué en une matière rigide, de préférence du plastique, à l'intérieur duquel est incorporé un filtre (4) et des essences naturelles (5), comprenant un fume-cigarette (3) et un bouchon (6), **caractérisé en ce que** ledit corps tubulaire (1) comprend deux secteurs de diamètre différent, l'un ayant un diamètre inférieur et de cote axiale inférieure, qui est configuré comme un prolongement du corps (1), en formant un col destiné à recevoir extérieurement le fume-cigarette (3) situé dans sa zone externe, fabriqué en une matière molle, de cote axiale coïncidente avec la cote axiale de la zone (2) et de diamètre extérieur à son tour coïncident avec le corps général (1), de telle manière qu'après le montage du fume-cigarette (3) sur le corps général, cet ensemble adopte une configuration et des dimensions similaires à celles d'une cigarette conventionnelle avec fume-cigarette ; et **en ce que** dans la zone du corps tubulaire (1) proche du bouchon (6), on a prévu l'incorporation d'un autre filtre (7).
